# EUROPÄISCHE PATENTANMELDUNG

(11) **EP 0 710 642 A2**
(43) Veröffentlichungstag der Anmeldung: **08.05.1996**
(21) Anmeldenummer: 96100322.5
(22) Anmeldetag: 22.02.1993
(51) Int. Cl.: C07C 49/327, C07C 45/63, C07D 249/08

(54) **Halogenketone**

(30) Priorität: 05.03.1992 DE 4206917
(62) Teilanmeldung aus: 93102759.3
(71) Anmelder: BAYER AG, D-51368 Leverkusen (DE)
(72) Erfinder: Böhm, Stefan, Dr., D-47800 Krefeld (DE); Marhold, Albrecht, Dr., D-51373 Leverkusen (DE)

(57) **Zusammenfassung**

Neue Halogenketone der Formel 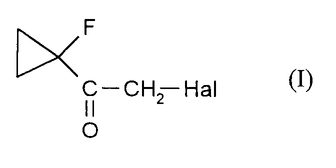
in welcher
Hal für Chlor oder Brom steht,
und ein Verfahren zu deren Herstellung.

## Beschreibung

Die vorliegende Erfindung betrifft neue Halogenketone und ein Verfahren zu deren Herstellung.

Es ist bereits bekannt geworden, daß sich 1-Fluorcyclopropyl-methyl-keton herstellen laßt, indem man 2-Acetyl-2-chlor-4-butanolid mit Kaliumfluorid behandelt, das dabei entstehende 2-Acetyl-1-fluor-4-butanolid mit Bromwasserstoff umsetzt und das sich dabei bildende 5-Chlor-3-fluor-pentan-2-on mit Kaliumfluorid cyclisiert (vergl. Synthesis 1977, 189-191). Diese Synthese kann durch das folgende Formelschema veranschaulicht werden: 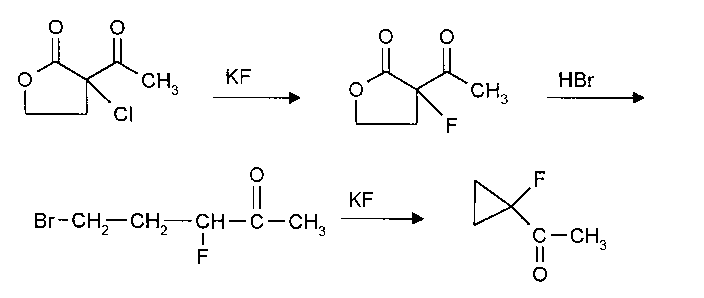
Eine Halogenierung des 1-Fluorcyclopropyl-methyl-ketons an der Methylgruppe wurde bisher aber noch nicht beschrieben.

Es wurden nun neue Halogenketone der Formel 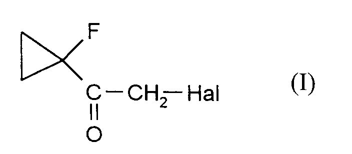
in welcher
Hal für Chlor oder Brom steht,
gefunden.

Weiterhin wurde gefunden, daß sich Halogenketone der Formel (I) herstellen lassen, indem man 1-Fluor-cyclopropyl-methyl-keton der Formel 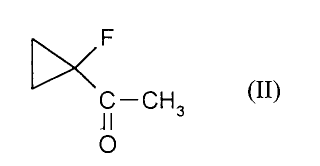
mit Chlorierungs- oder Bromierungsmitteln in Gegenwart eines Verdünnungsmittels umsetzt.

Bei der Durchführung des obigen Verfahrens kommen alle für derartige Umsetzungen üblichen Chlorierungs- und Bromierungsreagenzien in Betracht. Vorzugsweise verwendbar sind Sulfurylchlorid, Sulfurylbromid und Brom.

Als Verdünnungsmittel kommen bei der Herstellung der Halogenketone der Formel (I) nach dem obigen Verfahren alle für derartige Umsetzungen üblichen organischen Solventien in Frage. Vorzugsweise verwendbar sind halogenierte aliphatische Kohlenwasserstoffe, wie Methylenchlorid, Chloroform und Tetrachlorkohlenstoff.

Die Temperaturen können bei dem obigen Verfahren zur Herstellung von Halogenketonen der Formel (I) innerhalb eines bestimmten Bereiches variiert werden. Im allgemeinen arbeitet man bei Temperaturen zwischen -10°C und +60°C, vorzugsweise zwischen 0°C und +40°C.

Bei der Durchführung des obigen Verfahrens zur Herstellung von Halogenketonen der Formel (I) arbeitet man im allgemeinen unter Normaldruck. Es ist aber möglich, unter erhöhtem oder vermindertem Druck zu arbeiten.

Bei der Durchführung des obigen Verfahrens zur Herstellung von Halogenketonen der Formel (I) setzt man auf 1 Mol an 1-Fluorcyclopropyl-methyl-keton der Formel (II) im allgemeinen eine stöchiometrische Menge oder auch einen Überschuß an Chlorierungs- oder Bromierungsmittel ein. Die Aufarbeitung erfolgt nach üblichen Methoden. Im allgemeinen geht man so vor, daß man das Reaktionsgemisch mit Wasser versetzt, die Phasen trennt, und die organische Phase wäscht, trocknet und einengt. Der verbleibende Rückstand wird einer Destillation unter vermindertem Druck unterworfen.

Das 1-Fluorcyclopropyl-methyl-keton der Formel (II) ist bekannt (vgl. Synthesis 1977, 189-191).

Die Halogenketone der Formel (I) sind wertvolle Zwischenprodukte zur Synthese von Wirkstoffen mit fungiziden Eigenschaften (vergl. EP-OS 0 436 348 und EP-OS 0 297 345). So läßt sich zum Beispiel die Verbindung der Formel 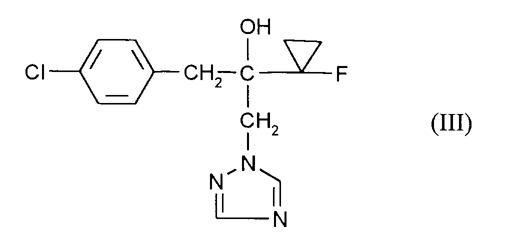
herstellen, indem man Halogenketone der Formel (I) mit 4-Chlorbenzyl-magnesium-chlorid der Formel 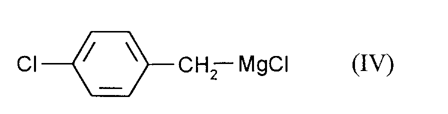
in Gegenwart eines Verdünnungsmittels umsetzt und die dabei anfallenden Propanol-Derivate der Formel 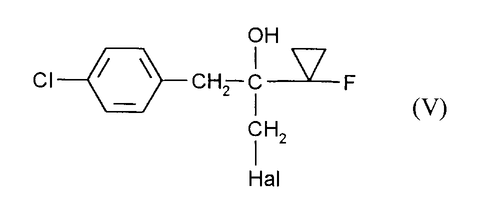
in welcher
Hai die oben angegebene Bedeutung hat,
mit 1,2,4-Triazol in Gegenwart eines Verdünnungsmittels und gegebenenfalls in Gegenwart eines Säurebindemittels umsetzt.

Die Erfindung wird durch die folgenden Beispiele veranschaulicht.

### Herstellungsbeispiel

Herstellung von 1-Fluorcyclopropyl-chlormethyl-keton der Formel 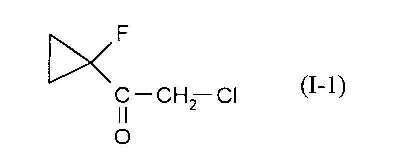
Unter Feuchtigkeitsausschluß werden 67,5 g (0,5 Mol) Sulfurylchlorid bei Raumtemperatur unter Rühren in ein Gemisch aus 40 g (0,4 Mol) 1-Fluor-cyclopropylmethyl-keton und 100 ml Methylenchlorid eingetropft. Nach beendeter Zugabe wird 6 Stunden bei Raumtemperatur nachgerührt. Anschließend gießt man das Reaktionsgemisch auf Eiswasser, trennt die Phasen, wäscht die organische Phase mit Wasser, trocknet über Calciumchlorid und zieht das Lösungsmittel unter vermindertem Druck ab. Der verbleibende Rückstand wird unter vermindertem Druck destilliert. Man erhält auf diese Weise 31,8 g (58% der Theorie) an 1-Fluorcyclopropyl-chlormethyl-keton in Form eines farblosen Öles.
Kp = 50°C / 22 mbar
¹H-NMR (200 MHz, CDCl₃, TMS-intern.)
δ = 1,46 ppm (s, 2H); 1,53 ppm (m, 2H), 4,65 ppm (d, 2H).
¹⁹F-NMR (200 MHz, CDCl₃, CFCl₃)
δ = 201,7 ppm (m).

### Verwendungsbeispiel

Herstellung der Verbindung der Formel

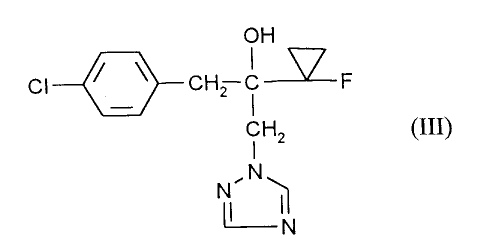

### a) Herstellung von 1-Chlor-2-(1-fluorcyclopropyl)-3-(4-chlorphenyl)-propan-2-ol der Formel

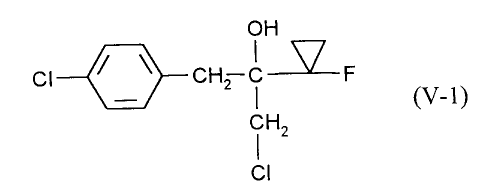

Eine Lösung von 37,3 g (0,25 Mol) 4-Chlor-benzylchlorid in 400 ml absolutem Diethylether wird bei Raumtemperatur in ein Gemisch aus 6,8 g (0,28 Mol) Magnesium-Spänen und 150 ml absolutem Diethylether getropft. Man erhitzt 30 Minuten unter Rückfluß und tropft die entstandene Lösung dann bei -78°C unter Rühren in eine Lösung von 34,1 g (0,25 Mol) 1-Fluorcyclopropyl-chlormethylketon in 250 ml absolutem Diethylether ein. Das Reaktionsgemisch wird 4 Stunden bei -78°C gerührt. Danach läßt man langsam auf 0°C erwärmen und tropft dann eine Lösung von 25 ml Essigsäure in 250 ml Diethylether hinzu. Das entstandene Reaktionsgemisch wird auf 1000 ml Wasser gegossen. Die organische Phase wird abgetrennt, mit wäßriger Natriumhydrogensulfit-Lösung und mit Wasser gewaschen, über Natriumsulfat getrocknet und unter vermindertem Druck eingeengt. Man erhält auf diese Weise 48,7 g (74% der Theorie) an 1-Chlor-2-(1-fluorcyclopropyl)-3-(4-chlorphenyl)propan-2-ol in Form eines oligen Produktes.

### b) Herstellung von 1-(4-Chlorphenyl)-2-(1-fluorcyclopropyl)-3-(1,2,4-triazol-1-yl)-propan-2-ol der Formel

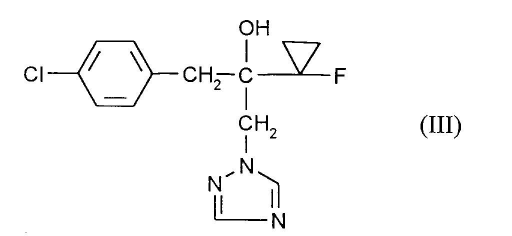

Unter Stickstoffatmosphäre werden 27,6 g (0,4 Mol) 1,2,4-Triazol und 33,6 g (0,3 Mol) Kalium-tert.-butylat in 100 ml absolutem Dimethylformamid vorgelegt und auf 80°C erwärmt. Bei dieser Temperatur tropft man unter Rühren eine Lösung von 26,3 g (0,1 Mol) 1-Chlor-2-(1-fluor-cyclopropyl)-3-(4-chlorphenyl)-propan-2-ol in 50 ml absolutem Dimethylformamid hinzu. Es wird 6 Stunden bei 100°C nachgeführt und dann durch Abziehen des Verdünnungsmittel unter vermindertem Druck eingeengt. Man nimmt den Rückstand in Essigester auf, wäscht mit Wasser und zieht nach dem Trocknen über Natriumsulfat das Lösungsmittel unter vermindertem Druck ab. Das verbleibende Produkt wird über eine Kieselgel-Säure mit Chloroform als Laufmittel chromatographiert. Man erhält auf diese Weise 8,9 g (35% der Theorie) an 1-(4-Chlorphenyl)-2-(1-fluor-cyclopropyl)-3-(1,2,4-triazol-1-yl)-propan-2-ol in Form einer Festsubstanz.

## Patentansprüche

1. Halogenketone der Formel 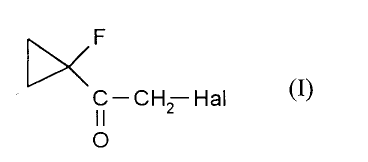 in welcher
Hal für Chlor oder Brom steht.

2. Verfahren zur Herstellung von Halogenketonen der Formel 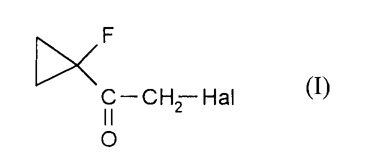 in welcher
Hal für Chlor oder Brom steht,
dadurch gekennzeichnet, daß man 1-Fluorcyclopropyl-methyl-keton der Formel 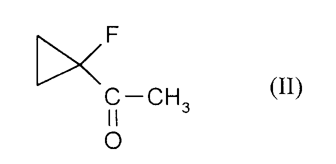 mit Chlorierungs- oder Bromierungsmitteln in Gegenwart eines Verdünnungsmittels umsetzt.
